(19) Europäisches Patentamt
European Patent Office
Office européen des brevets

(11) Numéro de publication: 0 253 700 B1

(12) # FASCICULE DE BREVET EUROPEEN

(45) Date de publication du fascicule du brevet: **29.08.90**

(51) Int. Cl.[5]: **C07B 41/02**, C07C 233/00, C07D 307/32

(21) Numéro de dépôt: **87401404.6**

(22) Date de dépôt: **22.06.87**

(54) **Procédé d'hydrogénation asymétrique de composés carbonylés et composés obtenus.**

(30) Priorité: **23.06.86 FR 8609024**

(43) Date de publication de la demande: **20.01.88 Bulletin 88/3**

(45) Mention de la délivrance du brevet: **29.08.90 Bulletin 90/35**

(84) Etats contractants désignés: **AT BE CH DE ES FR GB GR IT LI LU NL SE**

(56) Documents cités:
**EP-A- 0 136 210**
**GB-A- 2 114 134**

**JOURNAL OF THE CHEMICAL SOCIETY - CHEMICAL COMMUNICATIONS, no. 24, 1984, pages 1641-1643; K. TANI et al.: "Preparation of new chiral peralkyldiphosphines as efficient ligands for catalytic asymmetric hydrogenation of alpha-dicarbonyl compounds"**

(73) Titulaire: **NORSOLOR S.A., Tour Aurore Place des Reflets, F-92080 Paris la Défense Cédex 5(FR)**

(72) Inventeur: **Mortreux, André, 17, rue Gambetta, F-59510 Hem(FR)**
Inventeur: **Petit, Francis, 27, Allée de la Clairière, F-59650 Villeneuve d'Ascq(FR)**
Inventeur: **Karim, Abdallah Angle Yacoub El Mansour, Rue Allah-el-Fassi Immeuble Jawahir, appt. 11 Marrakech(MA)**

(74) Mandataire: **Chaillot, Geneviève, Cabinet CHAILLOT 21, avenue Louise de Bettignies, F-92700 Colombes(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 253 700 B1

ACTORUM AG

## Description

La présente invention a trait aux synthèses énantiosélectives de composés organiques, et elle se rapporte, plus particulièrement, à l'hydrogénation asymétrique de fonctions carbonyle, catalysée par des complexes de métaux de transition comportant des ligands phosphorés chiraux.

Si la réduction catalysée en phase homogène par des complexes de type Wilkinson, $Rh[PPh_3]_3C1$, s'avère très performante en synthèse asymétrique d'alpha-amino-acides à partir de précurseurs oléfiniques (H.B. Kagan, "Asymmetric synthesis using organometallic catalysts. Comprehensive organometallic chemistry, G. Wilkinson Ed. (1982) Vol 8, 463. Pergamon Press, London.), elle est cependant inopérante lors de la synthèse d'alcools chiraux à partir de cétones.

D'autre part, il est bien connu, en catalyse asymétrique, que les différents types de coordinats ou ligands s'avèrent spécifiques d'un composé de départ (substrat) donné.

On connaît, par la demande de brevet britannique GB-A 2 114 134, des phosphines chirales de formule:

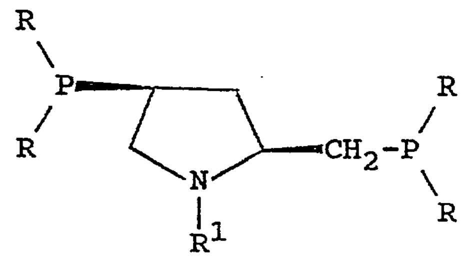

dans laquelle R représente aryle et $R^1$ peut représenter $-P-R^2$, $R^2$ représentant aryle, ces phosphines étant utilisées dans des catalyseurs pour des hydrogénations asymétriques, notamment de la dihydro-4,4 diméthyl-2,3 furannedione en R-(α-hydroxy β,β-diméthyl γ-butyrolactone).

Il a maintenant été découvert que l'hydrogénation asymétrique de composés carbonylés pouvait être envisagée avec d'excellents résultats, grâce à l'emploi de ligands obtenus en une seule étape à partir d'alpha-aminoalcools chiraux commerciaux ou issus d'alpha-aminoacides naturels, de tels ligands ayant déjà été décrits dans la demande de brevet européen EP-A 136 210 pour d'autres synthèses (comme l'hydrogénation asymétrique de dehydroaminoacides), et par M. Petit, A. Mortreux, F. Petit, G. Buono, G. Pfeiffer dans "Nouv. J. chim. 10 (7) (1983) 593".

Par ailleurs, il a été découvert, de façon surprenante, que le choix d'un milieu réactionnel dans lequel le substrat est soluble alors que le produit final ne l'est pas, permet, d'une part, d'atteindre des rendements optiques élevés, et, d'autre part, de travailler avec des rapports molaires substrat/métal catalyseur très élevés (pouvant aller jusqu'à 5000), ce qui n'avait jamais pu être effectué jusqu'ici. Ainsi, ces rapports sont de 50 et de 200 dans le cas des hydrogénations asymétriques de fonction carbonyle décrites respectivement par K. Yamamoto et al. et par K. Tani et al, la valeur de 200 représentant la limite supérieure que l'on pouvait atteindre jusqu'à maintenant.

La présente invention a pour objet un procédé d'hydrogénation asymétrique de composés carbonylés, ladite hydrogénation s'effectuant sur les fonctions carbonyle desdits composés et en présence, d'une part, d'au moins un complexe de métal de transition de formule:

MZq

dans laquelle:

– M est un métal du Groupe VIII de la Classification Périodique;

– Z représente un ou plusieurs parmi les atomes et molécules capables de complexer le métal M; et

– q est le degré de coordination du métal M,

et, d'autre part, d'au moins un ligand phosphoré chiral, caractérisé par le fait qu'on choisit, comme ligand phosphoré chiral, un composé représenté par la formule (I):

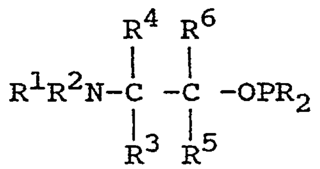

(I)

formule dans laquelle:

– R représente un radical hydrocarboné choisi parmi les radicaux alkyle, linéaires ou ramifiés, cycloalkyle ou aryle;

– $R^1$ représente un hydrogène, un radical hydrocarboné ou un reste $-PR^2$;

– $R^2$ représente un hydrogène ou un radical hydrocarboné;

– $R^3$, $R^4$, nécessairement différents, sont choisis parmi l'atome d'hydrogène et les radicaux hydrocarbonés éventuellement porteurs d'au moins une fonction choisie parmi les fonctions alcool, thiol, thioéther, amine, imine, acide, ester, amide et éther; et

– $R^5$ et $R^6$ sont choisis parmi l'atome d'hydrogène et les radicaux hydrocarbonés éventuellement fonctionnalisés;

– l'un des radicaux $R^3$ et $R^4$ pouvant porter une fonction $-OPR_2$ ou $-NPR_2$, $R^5$ et $R^6$ étant dans ce cas tous deux égaux à hydrogène lorsque $R^1$ représente $-PR_2$,

– $R^2$ et $R^3$, et les atomes d'azote et de carbone qui les portent respectivement pouvant former ensemble un hétérocycle à 5 ou 6 chaînons; ou bien $R^2$ et $R^5$, les atomes d'azote et de carbone qui les portent respectivement et l'atome de carbone intermédiaire pouvant former ensemble un hétérocycle à 5 ou 6 chaînons.

Cette famille de ligands se décompose en trois sous-familles selon le nombre de radicaux dihydrocarbyl-phosphine($-PR_2$) présents, à savoir les chélates monodentés (dans lesquels $R^1$ ne signifie pas $-PR^2$ et $R^3$ ne comporte pas de reste $-OPR_2$ ou $-NPR_2$); les chélates bidentés (pour lesquels, ou bien $R^1$ représente $-PR_2$ auquel cas $R^3$ et $R^4$ ne portent pas de fonction $-OPR_2$ ou $-NPR_2$, ou bien $R^3$ ou $R^4$ comporte un reste $-OPR_2$ ou $-NPR_2$, auquel cas $R^1$ ne peut représenter $-PR_2$ et les chélates tridentés (pour lesquels $R^1$ représente un reste $-PR_2$ et $R^3$ comporte un reste $-OPR_2$ ou $-NPR_2$).

Comme radiaux alkyle pour R, on citera les radicaux alkyle en $C_1$ -$C_{12}$, notamment en $C_1$ -$C_6$, par exemple les radicaux méthyle, éthyle, isopropyle et tertiobutyle. Comme radicaux cycloalkyle pour R, on mentionnera les radicaux cyclopentyle et cyclohexyle. Comme radicaux aryle pour R, on citera le radical phényle. On préfère, pour R, les radicaux alkyle et cycloalkyle.

Comme radical hydrocarboné pour $R^1$ ou $R^2$, on pourra mentionner les radicaux alkyle, notamment en $C_1$ -$C_{12}$, par exemple le radical méthyle.

Des exemples de radicaux hydrocarbonés pour $R^3$ ou $R^4$ sont, notamment, les radicaux méthyle, isopropyle, néobutyle, isobutyle, isoamyle, hydroxyméthyle, n-hydroxy-éthyle, iso-hydroxyéthyle, n-aminobutyle, méthylène-4 imidazolyle, N-n-propylguanidyle, éthanoyle, acétamidoyle, n-propionyle, n-propionamidoyle, phényle, benzyle, p-hydroxybenzyle, méthylène-3 indolyle, mercaptométhyle, méthylthioéthyle ou $NH_2$-C(=NH)-NH-$(CH_2)_3$-.

Comme radicaux hydrocarbonés pour $R^5$ ou $R^6$, on mentionnera notamment les radicaux alkyle en $C_1$-$C_{12}$.

Le procédé d'obtention de ces ligands de formule (I) est décrit dans la demande de brevet européen EP-A 136 210. En ce qui concerne le complexe MZq, on peut citer comme métaux M couramment utilisables, le fer, le nickel, le cobalt, le rhodium, le ruthénium, l'irridium, le palladium et le platinie; comme atomes ou molécules Z couramment utilisables, le monoxyde de carbone, les halogènes, l'éthylène, le norbornadiène, le cyclooctadiène et l'acétylacétone. Le degré de coordination, q, peut être couramment compris entre 2 et 6 inclusivement selon le métal M et/ou les ligands Z utilisés.

Le cas échéant, la réaction d'hydrogénation selon l'invention peut être effectuée en présence d'au moins un agent capable de capter un ligand Z du constituant MZq, cet agent pouvant être soit un acide possédant un anion $A^-$ peu coordinant et stériquement encombré ou un sel métallique d'un tel acide, soit une quantité d'électricité mise en jeu par électrolyse à potentiel cathodique imposé. Par anion $A^-$ peu coordinant et stériquement encombré, on entend notamment les anions perchlorate, tétrafluoroborate, tétraphénylborate et hexafluorophosphate. Les sels métalliques utilisables sont principalement ceux d'argent et de thallium.

Cette réaction de synthèse énantiosélective peut être effectuée en présence d'au moins un activateur choisi parmi les dérivés aluminiques de formule $AlR'_nX_{3-n}$, dans laquelle n vaut de 0 à 3, X est un atome d'halogène et R' est radical alkyle ayant de 1 à 12 atomes de carbone.

Le complexe MZq et le ligand de formule (I) sont généralement dans un rapport molaire: ligand/complexe compris entre 1 et 10. Le complexe MZq et le dérivé aluminique précité sont dans un rapport molaire: dérivé aluminique/complexe compris entre 0,1 et 10. Le complexe MZq et l'agent capable de capter au moins un ligand Z sont généralement dans un rapport molaire: agent/complexe inférieur ou égal à q, lorsque cet agent est un acide ou sel.

La réaction d'hydrogénation est généralement effectuée à une température comprise entre -20°C et 200°C, pendant une durée comprise entre 5 minutes et 24 heures environ, et sous une pression comprise entre 1 et 200 bars.

Par ailleurs, le rapport molaire du substrat (composé de départ) au métal M catalyseur peut atteindre des valeurs élevées de 5000, voire de 10000 ou davantage. Pour pouvoir travailler avec ces valeurs élevées, on utilise le mode de réalisation particulier suivant:

On effectue l'hydrogénation dans un milieu (solvant ou mélange de solvants) dans lequel le substrat est sensiblement soluble mais dans lequel le produit de la réaction est insoluble ou sensiblement insoluble, et de ce fait, précipite au fur et à mesure qu'il se forme.

Pour l'obtention d'un meilleur excès énantiomérique, on peut mentionner, comme solvants, les solvants hydrocarbonés aromatiques, tels que le benzène, le toluène et le xylène, les alcools aliphatiques, tels

que l'éthanol, et les éthers aliphatiques ou cycloaliphatiques, en particulier l'éther éthylique, le dioxanne et le tétrahydrofuranne, et les mélanges compatibles de ces solvents.

Parmi les composés de départ carbonylés mis en jeu dans cette réaction d'hydrogénation,on peut mentionner les α-cétoamides, tels que le N-benzylbenzoylformamide ($PhCOCONHCH_2Ph$) ou le N-benzyl hydroxy-4 benzoylformamide, aisément synthétisés par double carbonylation d'halogénures d'aryle catalysée par des complexes palladiés ; les composés α-dicarbonylés comme l'oxo-2-diméthyl-3,3-butanolide-1,4 (pentoyllacetone) et les α-cétoesters.

Pour mieux faire comprendre l'objet de la présente invention, on va en décrire maintenant plusieurs exemples de réalisation.

Dans le tableau I ci-après, est indiqué la nomenclature des ligands mis en oeuvre.

TABLEAU I: Nomenclature des ligands utilisés.

| | | |
|---|---|---|
| 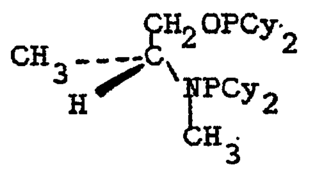 | CyAlaNOP | (S)-1-O-dicyclohexylphosphino)-2-N-méthyl-N-dicyclohexylphosphino-propane |
| 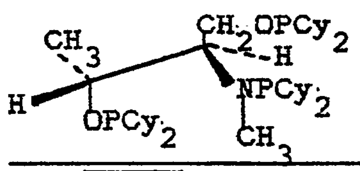 | CyThréoNOP | (2R,3R)-1,3-bis-O-dicyclohexylphosphino-2-N-méthyl-N-dicyclohexylphosphino-butane |
| 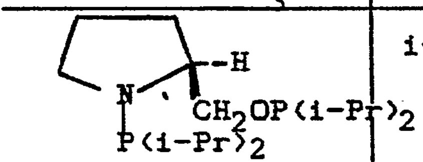 | i-PrProNOP | (S)-N-diisopropylphosphino-2-diisopropylphosphinoxyméthyl-pyrrolidine |
| 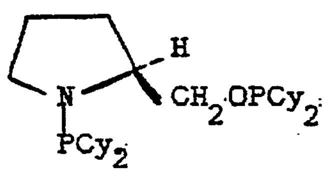 | CyProNOP | (S)-N-dicyclohexylphosphino-2-dicyclohexylphosphinoxyméthyl-pyrrolidine |
| 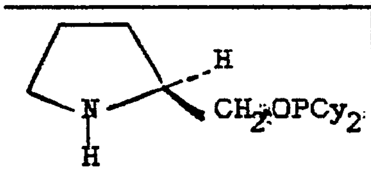 | CyProNHOP | (S)-2-dicyclohexylphosphinoxyméthyl-pyrrolidine |
| 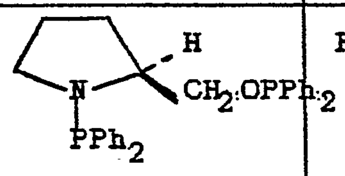 | ProNOP | (S)-N-diphénylphosphino-2-diphénylphosphinoxy-méthyl-pyrrolidine |
| 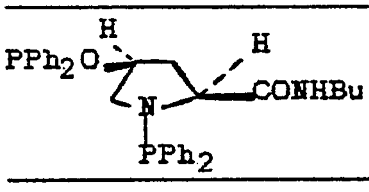 | Pro-BuNOP | (2S,4R)-N-diphénylphosphino-4-diphénylphosphino-2-n-butyl-formamide |

I - PREPARATION DES LIGANDS

a) Préparation du ligand CyProNOP

Dans un ballon de 250 $cm^3$, on introduit 2,53 g (0,025 mole) de prolinol et 14 $cm^3$ (0,1 mole) de $NEt_3$ en solution dans 50 $cm^3$ de benzène anhydre. 11,64 g de $PCy_2Cl$ en solution dans 50 $cm^3$ du même solvant sont ajoutés goutte à goutte à 0°C. Le mélange réactionnel est ensuite laissé sous agitation pendant 12 heures. La solution est ensuite filtrée pour la débarrasser du chlorhydrate de triéthylamine, et le solvant chassé sous vide. Le ligand est purifié par chromatographie sur gel de silice (éluant : acétate d'éthyle/$NEt_2H$ dans un rapport en volume 98/2).

b) Les autres ligands sont préparés selon le même protocole expérimental

II - HYDROGENATION CATALYTIQUE SELON LA PRESENTE INVENTION DE DIVERS COMPOSES CARBONYLES.

a) Mode opératoire général

On met en solution, dans 15 $cm^3$ de benzène anhydre, $6{,}6.10^{-5}$ mmole de ligand dans un tube sous azote.

14,8 mg ($3.10^{-5}$ mole) de complexe $[Rh(cyclooctadiène)Cl]_2$ sont pesés dans un autre tube et la solution de ligand y est ajoutée par tube de transfert. Le tout est laissé sous agitation pendant 15 minutes.

Dans un réacteur en verre thermostaté (25°C), on introduit $1{,}2.10^{-2}$ mole du composé de départ en solution dans 15 $cm^3$ de benzène sous atmosphère d'hydrogène après plusieurs purges (hydrogène-vide). La solution catalytique est ensuite transférée dans le réacteur, et l'évolution de la réaction est suivie par lecture du volume d'hydrogène consommé.

Le produit final est ensuite isolé du milieu réactionnel.

b) Obtention du produit final

1 - Obtention du (S)-N-benylmandélamide à partir du N-benzylbenzoylformamide

En fin de réaction, la solution de benzène est filtrée, et le précipité produit de la réaction est lavé par deux fois 10 $cm^3$ de benzène froid. Le rendement est de l'ordre de 95-100%.

Le pouvoir rotatoire spécifique $\alpha_D^{25}$ , est mesuré sur une solution de trichlorométhane de concentration 1,09g/100cm³. L'excès énantiomérique est calculé à partir de l'$\alpha_D^{25}$ du produit pur qui est de +79,9° (voir Tableau II).

2 - Obtention de l'hydroxy-2 diméthyl-3,3-butanolide-1,4 à partir de l'oxo-2-diméthyl-3,3-butanolide-1,4 (pentoyllactone)

En fin de réaction, le solvant est évaporé et le résidu, distillé sous vide. Le rendement est de l'ordre de 90-100%.

Le pouvoir rotatoire spécifique $\alpha_D^{25}$ est mesuré sur une solution aqueuse de concentration 2,05 g/100 $cm^3$. L'excès énantiomérique est calculé à partir de l'$\alpha_D^{25}$ du produit pur, qui est de -50,7°.

On a résumé dans les tableaux II à IV ci-après un certain nombre d'exemples d'hydrogénation asymétrique selon l'invention, exécutée selon le mode opératoire général décrit au paragraphe IIa), le tableau IV illustrant l'importance du choix du solvant (le produit de réduction du substrat N-benzylbenzoylformamide est soluble dans l'éthanol, et non dans le benzène).

## Tableau II

Hydrogénation asymétrique de composés carbonylés en présenc d'entités [Rh-L] (L= ligand).

| Composé de départ ou substrat (S) | Ligand ( L ) | Solvant | Durée de la réaction | Rendement ( % ) | Excès énantiomérique (%) | Configuration |
|---|---|---|---|---|---|---|
| $PhCOCONHCH_2Ph$ | CyProNOP | Benzène | 6 heures | 95 | 85,6 | S |
| " | " | " | " | 90 | 85,5[a] | S |
| " | " | " | " | 98 | 83[b] | S |
| " | " | " | " | 98 | 86[c] | S |
| " | iPrProNOP | " | " | 97 | 77 | S |
| HO-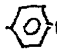-$COCONHCH_2Ph$ | CyProNOP | Benzène/ éthanol ( 2 / 1 ) | " | 100 | 66 | $\alpha_D^{20}$ = + 34,5 (C=1, éthanol) |
| 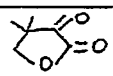 | CyProNOP | Benzène | 5 heures | 90 | 49,2 | R |
| " | " | " | 6 heures | 100 | 49,2 | R |
| " | iPrProNOP | " | 5 heures | 100 | 49,5 | R |
| " | CyProNOP | Tétrahydro-furanne | 5 heures | 100 | 46 | R |

<u>Conditions expérimentales générales:</u>
[S]/[Rh] = 200; [S] = 0,4 M; [Rh] = 2 x $10^{-3}$ M
[L] = 2,2 x $10^{-3}$ M
Quantité de solvant = 30 $cm^3$
Température = 25°C
Pression d'H2 = 1 bar

<u>Conditions expérimentales particulières</u>
(a) : [S]/[Rh] = 400 ; [Rh] = $10^{-3}$ M
(b) : [S]/[Rh] = 1000 ; [Rh] = 4 x $10^{-4}$ M
(c) : [S]/[Rh] = 5000 ; [Rh] = 8 x $10^{-5}$ M

## Tableau III

Hydrogénation du substrat N-benzylbenzoylformamide $PhCOCONHCH_2Ph$

| Ligand L | Rapport L / Rh | Vitesse d'hydrogénation ($cm^3$ d'hydrogène consommés par minute) | Temps de demi-réaction (mn) | Excès énantiomérique (%) | Configuration |
|---|---|---|---|---|---|
| CyProNOP | 1,1 | 2,3 | 43 | 85,6 | S |
| CyProNHOP | 1,1 | 0,6 | 200 | 48,7 | S |
| CyProNHOP | 2,2 | 0,65 | 200 | 49,7 | S |
| CyThréoNOP | 1,1 | 1,8 | 67 | 81,5 | S |
| CyAlaNOP | 1,1 | 2 | 55 | 74,4 | S |
| i-PrProNOP | 1,1 | 1,5 | 70 | 77 | S |
| ProNOP(a) | 1,1 | - | - | 46 | S |
| Pro-BuNOP(a) | 1,1 | - | - | 49,3 | S |

Conditions expérimentales :

[Rh] = $2 \times 10^3$ M ; [S]/[Rh] = 200
Solvant : Benzène (30 $cm^3$)
Température : 25°C
Pression : environ 1 bar
Rendement : supérieur à 90%
(a) : conditions réactionnelles différentes, soit: pression 40 bars; température 30°C et temps de réaction 12h

## Tableau IV

Influence du rapport molaire [S]/[Rh] lors de la réduction du N-benzylbenzoylformamide $PhCOCONHCH_2Ph$ avec le système "Rh, CyProNOP,Cl"

| Substrat (S) | Solvant | Temps de demi-réaction (mn) | Rendement (%) | Excès énantiomérique(%) | Configuration |
|---|---|---|---|---|---|
| 200 | Benzène | 55 | 95 | 85,6 | (S) |
| 400 | Benzène | 60 | 90 | 85,5 | (S) |
| 1000 | Benzène | 62 | 98 | 83 | (S) |
| 5000 | Benzène | 60 | 91 | 86 | (S) |
| 200 | Ethanol | 170 | 88 | 72 | (S) |
| 400 | Ethanol | 185 | 92 | 43,7 | (S) |

Conditions expérimentales :

[S] = 0,4 M
[L]/[Rh] = 1,1
Solvant : benzène (30 $cm^3$)
Température : 25°C
Pression : environ 1 bar

**Revendications**

1. Procédé d'hydrogénation asymétrique de composés carbonylés, ladite hydrogénation s'effectuant sur les fonctions carbonyle desdits composés et en présence, d'une part, d'au moins un complexe de métal de transition de formule:

MZq

dans laquelle:

– M est un métal du Groupe VIII de la Classification Périodique;

– Z représente au moins un ligand choisi parmi les atomes et les molécules capables de complexer le métal M; et

– q est le degré de coordination du métal M,

et, d'autre part, d'au moins un ligand phosphoré chiral, caractérisé par le fait qu'on choisit, comme ligand phosphoré chiral, un composé représenté par la formule (I):

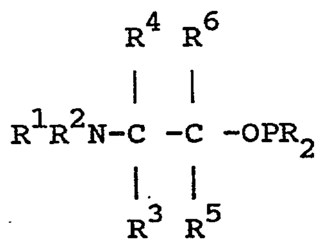

(I)

formule dans laquelle:

– R représente un radical hydrocarboné choisi parmi les radicaux alkyle, linéaires ou ramifiés, cycloalkyle ou aryle;

– $R^1$ représente un hydrogène, un radical hydrocarboné ou un reste $-PR_2$;

– $R^2$ représente un hydrogène ou un radical hydrocarboné;

– $R^3$, $R^4$, nécessairement différents, sont choisis parmi l'atome d'hydrogène et les radicaux hydrocarbonés éventuellement porteurs d'au moins une fonction choisie parmi les fonctions alcool, thiol, thioéther, amine, imine, acide, ester, amide et éther; et

– $R^5$ et $R^6$ sont choisis parmi l'atome d'hydrogène et les radicaux hydrocarbonés éventuellement fonctionnalisés;

– l'un des radicaux $R^3$ et $R^4$ pouvant porter une fonction $-OPR_2$ ou $-NPR_2$, $R^5$ et $R^6$ étant dans ce cas tous deux égaux à hydrogène lorsque $R^1$ représente $-PR_2$,

– $R^2$ et $R^3$ et les atomes d'azote et de carbone qui les portent respectivement pouvant former ensemble un hétérocycle à 5 ou 6 chaînons; ou bien $R^2$ et $R^5$, les atomes d'azote et de carbone qui les portent respectivement et l'atome de carbone intermédiaire pouvant former ensemble un hétérocycle à 5 ou 6 chaînons.

2. Procédé selon la revendication 1, caractérisé par le fait qu'on choisit, comme radical alkyle entrant dans la signification de R, un radical alkyle en $C_1$-$C_{12}$, et notamment en $C_1$-$C_6$, comme les radicaux méthyle, éthyle, isopropyle et tertiobutyle.

3. Procédé selon la revendication 1, caractérisé par le fait qu'on choisit, comme radical cycloalkyle entrant dans la signification de R, un radical cyclohexyle ou cyclopentyle.

4. Procédé selon la revendication 1, caractérisé par le fait qu'on choisit, comme radical aryle entrant dans la signification de R, un radical phényle.

5. Procédé selon l'une des revendications 1 à 4, caractérisé par le fait qu'on choisit, comme radical hydrocarboné entrant dans la définition de $R^1$ et $R^2$, un radical alkyle, notamment en $C_1$-$C_{12}$.

6. Procédé selon l'une des revendications 1 à 5, caractérisé par le fait qu'on choisit, comme radical hydrocarboné entrant dans la définition de $R^3$ et $R^4$, un radical méthyle, isopropyle, isobutyle, isoamyle, néobutyle, hydroxyméthyle, n-hydroxyéthyle, iso-hydroxyéthyle, n-aminobutyle, méthylène-4 imidazolyle, N-n-propylguanidyle, éthynoyle, acétamidoyle, n-propionyle, n-propionamidoyle, phényle, benzyle, p-hydroxybenzyle, méthylène-3 indolyle, méthylthioéthyle, mercaptométhyle ou $NH_2$-C(=NH)-NH$(CH_2)_3$-.

7. Procédé selon l'une des revendications 1 à 6, caractérisé par le fait qu'on choisit, comme radical hydrocarboné pour $R^5$ et $R^6$, un radical alkyle en $C_1$-$C_{12}$.

8. Procédé selon l'une des revendications 1 à 7, caractérisé par le fait qu'on choisit, comme métal M du complexe MZq, le fer, le nickel, le cobalt, le rhodium, le ruthénium, l'iridium, le palladium ou le platine.

9. Procédé selon l'une des revendications 1 à 8, caractérisé par le fait qu'on choisit, comme atome ou molécule Z du complexe MZq, le monoxyde de carbone, les halogènes, l'éthylène, le norbornadiène, le cyclooctadiène ou l'acétylacétone.

10. Procédé selon l'une des revendications 1 à 9, caractérisé par le fait que l'on choisit, pour q du complexe MZq, une valeur comprise entre 2 et 6 inclusivement.

11. Procédé selon l'une des revendications 1 à 10, caractérisé par le fait qu'on effectue l'hydrogénation en présence d'au moins un agent capable de capter un ligand Z du constituant MZq, cet agent pouvant être soit un acide possédant un anion $A^-$peu coordinant et stériquement encombré ou un sel métallique d'un tel acide, soit une quantité d'électricité mise en jeu par électrolyse à potentiel cathodique imposé.

12. Procédé selon l'une des revendications 1 à 11, caractérisé par le fait qu'on effectue l'hydrogénation en présence d'au moins un activateur choisi parmi les dérivés aluminiques de formule $AlR'_nX_{3-n}$, dans laquelle n vaut de 0 à 3, X est un atome d'halogène et R' est radical alkyle ayant de 1 à 12 atomes de carbone.

13. Procédé selon l'une des revendications 1 à 12, caractérisé par le fait qu'on travaille avec un rapport molaire: ligand de formule (I)/complexe MZq compris entre 1 et 10.

14. Procédé selon l'une des revendications 11 à 13, caractérisé par le fait qu'on travaille avec un rapport molaire: agent capable de capter un ligand Z/complexe MZq inférieur ou égal à q.

15. Procédé selon l'une des revendications 12 à 14, caractérisé par le fait qu'on travaille avec un rapport molaire: activateur/complexe MZq compris entre 0,1 et 10.

16. Procédé selon l'une des revendications 1 à 15, caractérisé par le fait qu'on conduit la réaction à une température comprise entre -20°C et 200°C.

17. Procédé selon l'une des revendications 1 à 16, caractérisé par le fait qu'on conduit la réaction pendant une durée comprise entre 5 minutes et 24 heures.

18. Procédé selon l'une des revendications 1 à 17, caractérisé par le fait qu'on travaille avec un rapport molaire: substrat/métal M catalyseur pouvant atteindre des valeurs élevées de l'ordre de 10 000, l'hydrogénation étant effectuée, pour pouvoir travailler avec ces valeurs élevées, dans un milieu dans lequel le substrat est sensiblement soluble mais dans lequel le produit de la réaction est insoluble ou sensiblement insoluble.

19. Procédé selon l'une des revendications 1 à 18, caractérisé par le fait que l'on effectue la réaction dans un solvant choisi parmi les solvants hydrocarbonés aromatiques, les alcools aliphatiques, les éthers aliphatiques et cycloaliphatiques et leurs mélanges.

20. Procédé selon l'une des revendications 1 à 19, caractérisé par le fait qu'on réalise l'hydrogénation de substrats choisis parmi les α-cétoamides, tels que le N-benzylbenzoylformanide ou le N-benzyl hydroxy-4 benzoyl-formamide; les composés α-dicarbonylés comme l'oxo-2-diméthyl-3,3-butanolide-1,4 et les α-cétoesters.

21. Composés hydrogénés obtenus par le procédé tel que défini à l'une des revendications 1 à 20.

**Claims**

1. A process for the asymmetric hydrogenation of carbonyl compounds, said hydrogenation being carried out on carbonyl groups of said compounds and in the presence, on the one hand, of at least one transition metal complex of formula:

MZq

in which

– M is a group VIII metal of the Periodic Table;

– Z represents at least one ligand chosen from amongst atoms and molecules capable of complexing the metal M; and

– q is the coordination number of the metal M,

and, on the other hand, of at least one chiral phosphorus-containing ligand, characterized by the fact that a compound represented by formula (I) is chosen as the chiral phosphorus-containing ligand:

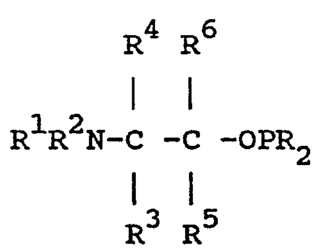

(I)

in which formula:

– R represents a hydrocarbon radical chosen from amongst straight-chain or branched alkyl, cycloalkyl or aryl radicals;

– $R^1$ represents a hydrogen, a hydrocarbon radical or a $-PR_2$ residue;

– $R^2$ represents a hydrogen or a hydrocarbon radical;

– $R^3$ and $R^4$, which are necessarily different, are chosen from amongst hydrogen atoms and hydrocarbon radicals optionally carrying at least one group chosen from amongst alcohol, thiol, thioether, amine, imine, acid, ester, amide and ehter groups; and

– $R^5$ and $R^6$ are chosen from amongst hydrogen atoms and optionally functionalized hydrocarbon radicals;
– it being possible for one of the radicals $R^3$ and $R^4$ to carry an -$OPR_2$ or -$NPR_2$ group, both $R^5$ and $R^6$ being, in this case, equal to hydrogen when $R^1$ represents -$PR^2$,
– it being possible for $R^2$ and $R^3$ and the nitrogen and carbon atoms which carry them respectively to together form a 5- or 6- membered heterocycle; or alternatively, it being possible for $R^2$ and $R^5$, the nitrogen and carbon atoms which carry them respectively and the intermediate carbon atom to together form a 5- or 6-membered heterocycle.

2. The process as claimed in claim 1, characterized by the fact that, as alkyl radical falling within the meaning of R, a $C_1$-$C_{12}$, and especially $C_1$-$C_6$, alkyl radical such as methyl, ethyl, isopropyl and tertbutyl, is chosen.

3. The process as claimed in claim 1, characterized by the fact that, as cycloalkyl radical falling within the meaning of R, a cyclohexyl or cyclopentyl radical is chosen.

4. The process as claimed in claim 1, characterized by the fact that, as aryl radicals falling within the meaning of R, a phenyl radical is chosen.

5. The process as claimed in one of claims 1 to 4, characterized by the fact that, as hydrocarbon radical falling within the definition of $R^1$ and $R^2$, an alkyl radical, especially $C_1$-$C_{12}$ alkyl radical, is chosen.

6. The process as claimed in one of claims 1 to 5, characterized by the fact that, as hydrocarbon radical falling within the definition of $R^3$ and $R^4$, a methyl, isopropyl, isobutyl, isoamyl, neobutyl, hydroxymethyl, n-hydroxyethyl, isohydroxyethyl, n-aminobutyl, 4-methyleneimidazolyl, N-n-propylguanidyl, ethanoyl, acetamidoyl, n-propionyl, n-propionamidoyl, phenyl, benzyl, para-hydroxybenzyl, 3-methyleneindolyl, methylthioethyl, mercaptomethyl or $NH_2$-C(=NH)-NH$(CH_2)_3$- is chosen.

7. The process as claimed in one of claims 1 to 6, characterized by the fact that, as hydrocarbon radical for $R^5$ and $R^6$, a $C_1$-$C_{12}$ alkyl radical is chosen.

8. The process as claimed in one of claims 1 to 7, characterized by the fact that, as metal M of the complex MZq, iron, nickel, cobalt, rhodium, ruthenium, iridium, palladium or platinum is chosen.

9. The process as claimed in one of claims 1 to 8, characterized by the fact that, as atom or molecule Z of the complex MZq, carbon monoxide, halogens, ethylene, norbornadiene, cyclooctadiene or acetylacetone is chosen.

10. The process as claimed in one of claims 1 to 9, characterized by the fact that a value between 2 and 6 inclusive is chosen for q of the complex MZq.

11. The process as claimed in one of claims 1 to 10, characterized by the fact that the hydrogenation is carried out in the presence of at least one agent capable of taking up a ligand Z from the ccnstituent MZq, it being possible for this agent to be either an acid having an anion $A^-$ which has a low complexing capacity and which is sterically hindered or a metal salt of such an acid, or a quantity of electricity applied by electrolysis at an applied cathode potential.

12. The process as claimed in one of claims 1 to 11, characterized by the fact that the hydrogenation is carried out in the presence of at least one activator chosen from amongst aluminum-containing derivatives of formula $AlR'_nX_{3-n}$, in which n has a value from 0 to 3, X is a halogen atom and R' is an alkyl radical containing from 1 to 12 carbon atoms.

13. The process as claimed in one of claims 1 to 12, characterized by the fact that the reaction is carried out using a molar ratio ligand of formula (I): complex MZq of between 1 and 10.

14. The process as claimed in one of claims 11 to 13, characterized by the fact that the reaction is carried out using a molar ratio agent capable of capturing a ligand Z: complex MZq less than or equal to q.

15. The process as claimed in one of claims 12 to 14, characterized by the fact that the reaction is carried out using a molar ratio activator: complex MZq of between 0.1 and 10.

16. The process as claimed in one of claims 1 to 15, characterized by the fact that the reaction is carried out at a temperature of between -20°C and 200°C.

17. The process as claimed in one of claims 1 to 16, characterized by the fact that the reaction is carried out during a period of time of between 5 minutes and 24 hours.

18. The process as claimed in one of claims 1 to 17, characterized by the fact that the reaction is carried out using a molar ratio substrate: catalyst metal M which may reach high values of the order of 10 000, the hydrogenation being carried out, in order to be able to work at these high values, in a medium in which the substrate is substantially soluble, but in which the reaction product is insoluble or substantially insoluble.

19. The process as claimed in one of claims 1 to 18, characterized by the fact that the reaction is carried out in a solvent chosen from amongst aromatic hydrocarbon solvents, aliphatic alcohols, aliphatic and alicyclic ehters and their mixtures.

20. The process as claimed in one of claims 1 to 19, characterized by the fact that hydrogenation is carried out, of substrates chosen from amongst α-keto amides such as N-benzylbenzoylformamide or N-benzyl-4-hydroxybenzoylformamide and α-dicarbonyl compounds such as 2-oxo-3,3-dimethyl -1,4-butanolide and α-keto esters.

21. Hydrogenated compounds obtained by the process as defined in one of claims 1 to 20.

## Patentansprüche

1. Verfahren zur asymmetischen Hydrierung von Carbonylverbindungen, wobei die genannte Hydrierung an den Carbonylfunktionen der genannten Verbindungen vorsichgeht, und zwar einerseits in Gegenwart von mindestens einem Komplex eines Übergangsmetalles der Formel

MZq,

in welcher M ein Metall der Gruppe VIII des Periodensystems ist, Z mindestens einen Liganden bedeutet, ausgewählt aus den Atomen und den Molekülen, die fähig sind mit dem Metall M Komplexe zu bilden, und q die Koordinationszahl des Metalles M ist, und anderseits mindestens einem chiralen phosphorhaltigen Liganden, dadurch gekennzeichnet, daß man als chiralen phosphorhaltigen Liganden eine durch die Formel (I) dargestellte Verbindung auswählt

$$\begin{array}{cccc} & R^4 & R^6 & \\ & | & | & \\ R^1R^2N- & C & -C & -OPR_2 \\ & | & | & \\ & R^3 & R^5 & \end{array} \qquad (I)$$

in welcher R einen Kohlenwasserstoffrest bedeutet, ausgewählt aus linearen oder verzweigten Alkyl-, Cycloalkyl- oder Arylresten; $R^1$ Wasserstoff, einen Kohlenwasserstoffrest oder einen Rest $-PR_2$ bedeutet; $R^2$ Wasserstoff oder einen Kohlenwasserstoffrest bedeutet; $R^3$, $R^4$, die notwendigerweise verschieden sind, ausgewählt sind aus Wasserstoff und den Kohlenwasserstoffresten, welche gegebenenfalls mindestens eine Funktion, ausgewählt aus Alkohol, Thiol, Thioäther, Amin, Imin, Säure, Ester, Amid und Äther tragen; und $R^5$ und $R^6$ ausgewählt sind aus Wasserstoff und den Kohlenwasserstoffresten, welche gegebenenfalls funktionalisiert sind; wobei einer der Reste $R^3$ und $R^4$ eine $-OPR_2$ oder $-NPR_2$ Funktion aufweisen kann, und in diesem Fall $R^5$ und $R^6$ beide Wasserstoff sind, wenn $R^1$ $-PR_2$ bedeutet, und $R^2$ und $R^3$ zusammen mit dem Stickstoffatom und dem Stickstoffatom und dem Kohlenstoff, der diese trägt, einen Heterocyclus mit 5 oder 6 Gliedern bilden können; oder auch $R^2$ und $R^5$, zusammen mit dem Stickstoffatom, dem Kohlenstoff, der diese trägt und dem dazwischenliegenden Kohlenstoffatom einen Heterocyclus mit 5 oder 6 Gliedern bilden können.

2. Verfahren nach Anspruch 1, dadurch gekennzeichnet, daß man als bei R angegebenen Alkylrest einen $C_1$-$C_{12}$ Alkylrest, insbesondere einen $C_1$-$C_6$-Alkylrest auswählt, wie den Methyl-Äthyl-, Isopropyl und tert. Butylrest.

3. Verfahren nach Anspruch 1, dadurch gekennzeichnet, daß man als bei R angegebenen Cycloalkylrest einen Cyclohexyl- oder Cyclopentylrest auswählt.

4. Verfahren nach Anspruch 1, dadurch gekennzeichnet, daß man als bei R angegebenen Arylrest einen Phenylrest auswählt.

5. Verfahren nach einem der Ansprüche 1 bis 4, dadurch gekennzeichnet, dass man als bei $R^1$ und $R^2$ angegebenen Kohlenwasserstoffrest insbesondere einen $C_1$-$C_{12}$ Alkylrest auswählt.

6. Verfahren nach einem der Ansprüche 1 bis 5, dadurch gekennzeichnet, daß man als bei $R^3$ und $R^4$ angegebenen Kohlenwasserstoffrest einen Methyl-, Isopropyl-, Isobutyl-, Isoamyl-, Neobutyl-, Hydroxymethyl-, n-Hydroxyäthyl-, Isohydroxyäthyl-, n-Aminobutyl-, 4-Methylenimidazolyl-, N-n-Propylguanidyl-, Äthanoyl-, Acetamidoyl-, n-Propionyl-, n-Propionamidoyl-, Phenyl-, Benzyl-, p-Hydroxybenzyl-, 3-Methylenindolyl-, Methylthioäthyl-, Mercaptomethyl- oder $NH_2$-C(=NH)-NH$(CH_2)_3$-Rest auswählt.

7. Verfahren nach einem der Ansprüche 1 bis 6, dadurch gekennzeichnet, daß man als Kohlenwasserstoffrest für $R^5$ und $R^6$ einen $C_1$-$C_{12}$- Alkylrest auswählt.

8. Verfahren nach einem der Ansprüche 1 bis 7, dadurch gekennzeichnet, daß man als Metall M des Komplexes MZq Eisen, Nickel, Cobalt, Rhodium, Ruthenium, Iridium, Palladium oder Platin auswählt.

9. Verfahren nach einem der Ansprüche 1 bis 8, dadurch gekennzeichnet, daß man als Atom oder Molekül Z Komplexes MZq Kohlenmonoxyd, die Halogene, Äthylen, Norbornadien, Cyclooctadien oder Acetylaceton auswählt.

10. Verfahren nach einem der Ansprüche 1 bis 9, dadurch gekennzeichnet, daß man für q des Komplexes MZq einen Wert zwischen einschließlich 2 und 6 auswählt.

11. Verfahren nach einem der Ansprüche 1 bis 10, dadurch gekennzeichnet, daß man die Hydrierung in Gegenwart mindestens eines Mittels, das fähig ist einen Liganden Z des Bestandteiles MZq einzufangen, wobei dieses Mittel entweder eine Säure ist, welche ein Anion $A^-$ aufweist, das wenig koordinativ und sterisch gehindert ist, oder ein Metallsalz einer solchen Säure oder eine durch Elektrolyse mit aufgeprägtem Kathodenpotential zur Wirkung gebrachte Elektrizitätsmenge ist.

12. Verfahren nach einem der Ansprüche 1 bis 11, dadurch gekennzeichnet, daß man die Hydrierung in Gegenwart von mindestens einem Aktivator, ausgewählt aus den Aluminiumderivaten der Formel $AlR'_nX_{3-n}$, in welcher n 0 bis 3 ist, X ein Halogenatom ist und R' einen Alkylrest mit 1 bis 12 C-Atomen bedeutet, durchführt.

13. Verfahren nach einem der Ansprüche 1 bis 12, dadurch gekennzeichnet, daß man mit einem molaren Verhältnis Ligand der Formel (I)/Komplex MZq zwischen einschließlich 1 und 10 arbeitet.

14. Verfahren nach einem der Ansprüche 11 bis 13, dadurch gekennzeichnet, daß man mit einem molaren Verhältnis Mittel, das fähig ist einen Liganden Z einzufangen/Komplex MZq unterhalb oder gleich q arbeitet.

15. Verfahren nach einem der Ansprüche 12 bis 14, dadurch gekennzeichnet, daß man mit einem molaren Verhältnis Aktivator/Komplex MZq zwischen einschließlich 0,1 und 10 arbeitet.

16. Verfahren nach einem der Ansprüche 1 bis 15, dadurch gekennzeichnet, daß man die Reaktion bei einer Temperatur zwischen einschließlich -20°C und 200°C durchführt.

17. Verfahren nach einem der Ansprüche 1 bis 16, dadurch gekennzeichnet, daß man die Reaktion während einer Dauer von einschließlich 5 min und 24 h durchführt.

18. Verfahren nach einem der Ansprüche 1 bis 17, dadurch gekennzeichnet, daß man mit einem molaren Verhältnis Substrat/Metall M Katalysator arbeitet, welches höhere Werte in der Größenordnung von 10 000 erreichen kann, wobei die Hydrierung, um mit diesen höheren Werten arbeiten zu können, in einem Milieu ausgeführt wird, in welchem das Substrat im wesentlichen löslich ist, in welchem jedoch das Reaktionsprodukt unlöslich oder im wesentlichen unlöslich ist.

19. Verfahren nach einem der Ansprüche 1 bis 18, dadurch gekennzeichnet, daß die Reaktion in einem Lösungsmittel, ausgewählt aus aromatischen Kohlenwasserstoff-Lösungsmitteln, aliphatischen Alkoholen, aliphatischen und cycloaliphatischen Äthern und ihren Mischungen, ausgeführt wird.

20. Verfahren nach einem der Ansprüche 1 bis 19, dadurch gekennzeichnet, daß man die Hydrierung der Substrate, ausgewählt aus den α-Ketoamiden, wie N-Benzylbenzoylformamid oder N-Benzyl-4-hydroxy-benzoylformamid, den α-Dicarbonylverbindungen, wie 2-Oxo-3,3-dimethyl-1,4-butanolid und den α-Ketoestern, durchführt.

21. Hydrierte Verbindungen erhalten nach dem in einem der Ansprüche 1 bis 20 definierten Verfahren.